# EUROPÄISCHE PATENTSCHRIFT

(11) **EP 3 502 085 B1**
(45) Veröffentlichungstag und Bekanntmachung des Hinweises auf die Patenterteilung: **11.03.2020**
(21) Anmeldenummer: 17209348.6
(22) Anmeldetag: 21.12.2017
(51) Int. Cl.: C07C 51/14, C07C 53/122, C07C 53/126, C07C 53/128, C07C 53/134, C07C 59/68, C07C 63/04

(54) **VERFAHREN ZUR DIREKTEN UMSETZUNG VON ALKENEN ZU CARBONSÄUREN**
METHOD FOR DIRECT CONVERSION OF ALKENES TO CARBOXYLIC ACIDS
PROCÉDÉ DE CONVERSION DIRECTE DES ALCÈNES EN ACIDES CARBOXYLIQUES

(43) Veröffentlichungstag der Anmeldung: 26.06.2019
(73) Patentinhaber: Evonik Operations GmbH, 45128 Essen (DE)
(72) Erfinder: SANG, Rui, 252000 Liaocheng City, Shandong Province (CN); KUCMIERCZYK, Peter, 44652 Herne (DE); DONG, Kaiwu, 236800 Bo Zhou (CN); JACKSTELL, Ralf, 10106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- EP-A1- 3 272 733
- EP-A1- 3 272 759
- EP-A1- 3 272 760
- WO-A1-2006/066975
- DE-A1- 19 621 967
- US-B2- 8 445 711

## Beschreibung

Die Erfindung betrifft ein Verfahren zur direkten Umsetzung von Alkenen zu Carbonsäuren.

Carbonsäuren werden bei der Herstellung von Polymeren, Pharmazeutika, Lösungsmitteln und Lebensmittelzusatzstoffen verwendet. Zu den Routen, welche zu Carbonsäuren führen, gehören im Allgemeinen die Oxidation von Kohlenwasserstoffen, Alkoholen oder Aldehyden, die oxidative Spaltung von Olefinen durch Ozonolyse, die Hydrolyse von Triglyceriden, Nitrilen, Estern oder Amiden, die Carboxylierung von Grignard- oder Organolithium-Reagenzien und die Halogenierung und anschließende Hydrolyse von Methylketonen in der Haloform-Reaktion.

WO 2006/066975 A1 offenbart die Herstellung von Carbonsäuren durch Hydroxycarbonylierung von Aryl- und Vinylbromiden.

Der Erfindung lag die Aufgabe zugrunde, ein Verfahren bereitzustellen, mit welchem Alkene direkt zu einer Carbonsäure umgesetzt werden können.

Unter "direktem Umsatz" ist im Rahmen dieser Anmeldung zu verstehen, dass die Reaktion in einem Schritt, also ohne Abtrennung oder Aufarbeitung oder dergleichen eines Zwischenproduktes erfolgt.

Hierbei ist nicht ausgeschlossen, dass sich im Verlauf der Reaktion intermediär Zwischenstufen ausbilden, welche direkt weiter umgesetzt werden.

Gelöst wird die Aufgabe durch ein Verfahren nach Anspruch 1.

Verfahren umfassend die Verfahrensschritte:
a) Zugabe eines Alkens;
b) Zugabe eines Komplexes, welcher eine Verbindung gemäß der Struktur (**1**) umfasst, sowie Pd,
   oder einer Verbindung gemäß der Struktur (**1**) und eine Substanz, welche Pd umfasst 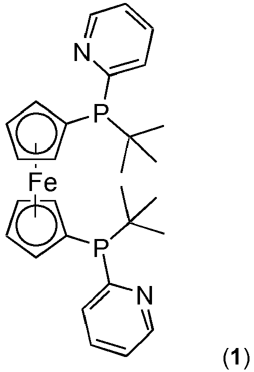
c) Zuführen von CO;
d) Erhitzen des Reaktionsgemisches, so dass das Alken zu einer Carbonsäure umgesetzt wird,
   wobei das Alken direkt zur Carbonsäure umgesetzt wird.

In einer Variante des Verfahrens ist die Substanz im Verfahrensschritt b) ausgewählt aus: PdCl₂, PdBr₂, Pd(acac)₂, Pd(dba)₂ (dba = Dibenzylidenaceton), PdCl₂(CH₃CN)₂.

In einer Variante des Verfahrens ist die Substanz im Verfahrensschritt b) Pd(acac)₂.

In einer Variante des Verfahrens umfasst das Verfahren den zusätzlichen Verfahrensschritt e):
e) Zugab von Essigsäure.

In einer Variante des Verfahrens umfasst das Verfahren den zusätzlichen Verfahrensschritt f):
f) Zugab von Wasser.

In einer Variante des Verfahrens umfasst das Verfahren den zusätzlichen Verfahrensschritt g):
g) Zugabe von p-Toluolsulfonsäure (PTSA).

In einer Variante des Verfahrens wird das Reaktionsgemisch im Verfahrensschritt d) auf eine Temperatur im Bereich von 80 °C bis 160 °C erhitzt.

In einer bevorzugten Variante des Verfahrens wird das Reaktionsgemisch im Verfahrensschritt d) auf eine Temperatur im Bereich von 100 °C bis 140 °C erhitzt.

In einer Variante des Verfahrens erfolgt das Zuführen von CO im Verfahrensschritt c) so, dass die Reaktion bei einem CO-Druck im Bereich von 20 bar bis 60 bar verläuft.

In einer bevorzugten Variante des Verfahrens erfolgt das Zuführen von CO im Verfahrensschritt c) so, dass die Reaktion bei einem CO-Druck im Bereich von 30 bar bis 50 bar verläuft.

Im Folgenden wird die Erfindung anhand von Ausführungsbeispielen näher erläutert.

### Variation des Liganden

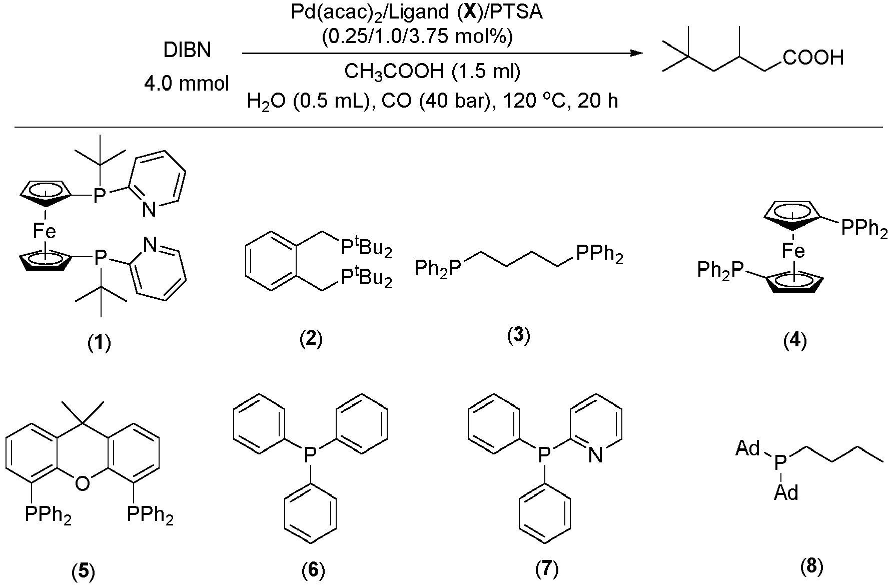

Ein 4-mL-Vial wurde mit [Pd(acac)₂] (3,05 mg, 0,25 mol-%), Ligand (X) (1,0 mol-%), p-Toluolsulfonsäure (28.5 mg, 3.75 mol-%) und einem im Ofen getrockneten Rührstab beschickt. Dann wird das Vial mit Septen (PTFE-beschichteter Styrol-Butadien-Kautschuk) und Phenolharzdeckel verschlossen. Das Vial wird dreimal evakuiert und mit Argon wieder befüllt. Mit einer Spritze wurden H₂O (0,5 ml), Essigsäure (1,5 ml) und Diisobuten (DIBN) (4,0 mmol) in das Vial gegeben. Das Vial wurde in eine Legierungsplatte gestellt, die in einen Autoklaven (300 ml) der Reihe 4560 von Parr Instruments unter Argonatmosphäre überführt wurde. Nach dreimaligem Spülen des Autoklaven mit CO wurde der CO-Druck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Dann wurde Isooctan (100 ul) als interner Standard zugegeben. Der Umsatz wurde durch GC-Analyse gemessen.

Der oben beschriebene Versuch wurde unter Variation des Liganden (**X**) mit **X** = **1** bis **8** durchgeführt.

Die Ergebnisse sind in der nachfolgenden Tabelle 1 zusammengestellt:

**Tabelle 1:**

| Ligand | Ausbeute (%) |
|---|---|
| (**1**)* | > 99 |
| (**2**) | 7 |
| (**3**) | 39 |
| (**4**) | 26 |
| (**5**) | 16 |
| (**6**) | 8 |
| (**7**) | 13 |
| (**8**) | 29 |

| | |
|---|---|
| * erfindungsgemäßes Verfahren | |

### Variation des Alkens

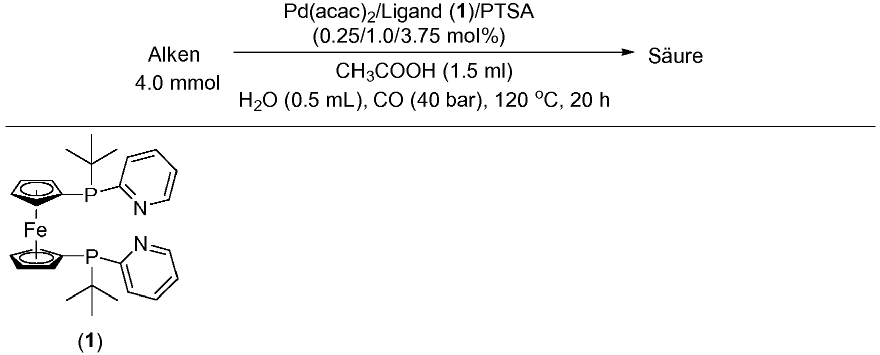

Ein 4-mL-Vial wurde mit [Pd(acac)₂] (3,07 mg, 0,25 mol.%), Ligand (**1**) (20,64 mg, 1,0 mol-%), p-Toluolsulfonsäure (28.5 mg, 3.75 mol%) und einem im Ofen getrockneten Rührstab beschickt. Dann wird das Vial mit Septen (PTFE-beschichteter Styrol-Butadien-Kautschuk) und Phenolharzdeckel verschlossen. Danach wird das Vial mit einer Nadel mit der Atmosphäre verbunden. Das Vial wird dreimal evakuiert und mit Argon wieder befüllt. Mit einer Spritze wurden H₂O (0,5 ml), Essigsäure (1,5 ml) und Alken (4,0 mmol) in das Vial gegeben. Das Vial wurde in eine Legierungsplatte gestellt, die in einen Autoklaven (300 ml) der Reihe 4560 von Parr Instruments unter Argonatmosphäre überführt wurde. Nach dreimaligem Spülen des Autoklaven mit CO wurde der CO-Druck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Dann wurde Isooctan (100 ul) als interner Standard zugegeben. Der Umsatz wurde durch GC-Analyse gemessen.

Der oben beschriebene Versuch wurde unter Variation des Alkens durchgeführt.

Die Ergebnisse sind in der nachfolgenden Tabelle 2 zusammengestellt:

**Tabelle 2:**

| Alken | Säure | Ausbeute |
|---|---|---|
| 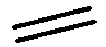 | 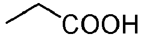 | > 99 % |
| 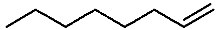 | 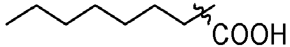 | 98 %, n/iso-Mischung |
| 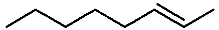 | 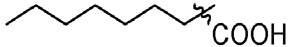 | 97 %, iso-Mischung |
| 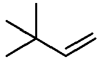 | 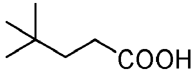 | 99 % |
| 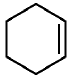 | 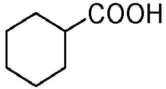 | 80 % |
| 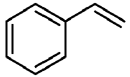 | 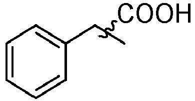 | 95 %, n/iso-Mischung |
| 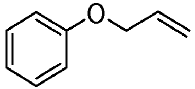 | 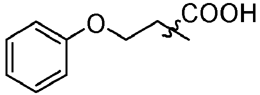 | 94 %, n/iso-Mischung |

Wie die Versuchsergebnisse zeigen, wird die Aufgabe durch ein erfindungsgemäßes Verfahren gelöst.

## Patentansprüche

1. Verfahren umfassend die Verfahrensschritte:
a) Zugabe eines Alkens;
b) Zugabe eines Komplexes, welcher eine Verbindung gemäß der Struktur (**1**) umfasst, sowie Pd,
oder einer Verbindung gemäß der Struktur (**1**) und eine Substanz, welche Pd umfasst 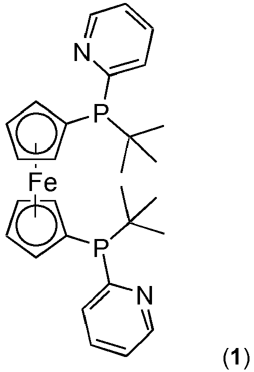
c) Zuführen von CO;
d) Erhitzen des Reaktionsgemisches, so dass das Alken zu einer Carbonsäure umgesetzt wird,
wobei das Alken direkt zur Carbonsäure umgesetzt wird.

2. Verfahren nach Anspruch 1,
wobei die Substanz im Verfahrensschritt b) ausgewählt ist aus:
PdCl₂, PdBr₂, Pd(acac)₂, Pd(dba)₂ (dba = Dibenzylidenaceton), PdCl₂(CH₃CN)₂.

3. Verfahren nach einem der Ansprüche 1 oder 2,
wobei das Verfahren den zusätzlichen Verfahrensschritt e) umfasst:
e) Zugab von Essigsäure.

4. Verfahren nach einem der Ansprüche 1 bis 3,
wobei das Verfahren den zusätzlichen Verfahrensschritt f) umfasst:
f) Zugab von Wasser.

5. Verfahren nach einem der Ansprüche 1 bis 4,
wobei das Verfahren den zusätzlichen Verfahrensschritt g) umfasst:
g) Zugabe von p-Toluolsulfonsäure.

6. Verfahren nach einem der Verfahrensschritte 1 bis 5,
wobei das Reaktionsgemisch im Verfahrensschritt d) auf eine Temperatur im Bereich von 80 °C bis 160 °C erhitzt wird.

7. Verfahren nach einem der Verfahrensschritte 1 bis 6,
wobei das Zuführen von CO im Verfahrensschritt c) so erfolgt, dass die Reaktion bei einem CO-Druck im Bereich von 20 bar bis 60 bar verläuft.

## Claims

1. Process comprising the process steps of:
a) addition of an alkene;
b) addition of a complex, comprising a compound according to structure (**1**) and also Pd,
or a compound according to structure (**1**) and a substance comprising Pd 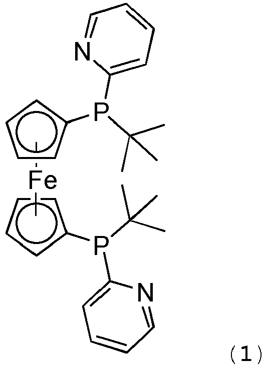
c) feeding in CO;
d) heating the reaction mixture such that the alkene is converted to a carboxylic acid,
wherein the alkene is directly converted to the carboxylic acid.

2. Process according to Claim 1,
wherein the substance in process step b) is selected from:
PdCl₂, PdBr₂, Pd(acac)₂, Pd(dba)₂ (dba = dibenzylideneacetone), PdCl₂(CH₃CN)₂.

3. Process according to either of Claims 1 and 2,
wherein the process comprises the additional process step e) :
e) addition of acetic acid.

4. Process according to any of Claims 1 to 3,
wherein the process comprises additional process step f):
f) addition of water.

5. Process according to any of Claims 1 to 4,
wherein the process comprises the additional process step g) :
g) addition of p-toluenesulfonic acid.

6. Process according to any of process steps 1 to 5, wherein the reaction mixture is heated to a temperature in the range from 80°C to 160°C in process step d).

7. Process according to any of process steps 1 to 6, wherein the CO is fed in in process step c) such that the reaction proceeds under a CO pressure in the range from 20 bar to 60 bar.

## Revendications

1. Procédé comprenant les étapes de procédé :
a) ajout d'un alcène ;
b) ajout d'un complexe, qui comprend un composé selon la structure (1), ainsi que du Pd,
ou d'un composé selon la structure (1) et d'une substance, qui comprend du Pd 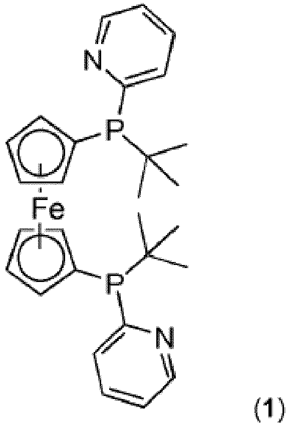
c) introduction de CO ;
d) chauffage du mélange réactionnel, de sorte que l'alcène est transformé en un acide carboxylique,
l'alcène étant directement transformé en l'acide carboxylique.

2. Procédé selon la revendication 1,
la substance dans l'étape de procédé b) étant choisie parmi :
PdCl₂, PdBr₂, Pd(acac)₂, Pd(dba)₂ (dba = dibenzylidèneacétone), PdCl₂(CH₃CN)₂.

3. Procédé selon l'une quelconque des revendications 1 et 2,
le procédé comprenant l'étape de procédé supplémentaire e) :
e) ajout d'acide acétique.

4. Procédé selon l'une quelconque des revendications 1 à 3,
le procédé comprenant l'étape de procédé supplémentaire f) :
f) ajout d'eau.

5. Procédé selon l'une quelconque des revendications 1 à 4,
le procédé comprenant l'étape de procédé supplémentaire g) :
g) ajout d'acide p-toluènesulfonique.

6. Procédé selon l'une quelconque des étapes de procédé 1 à 5,
le mélange réactionnel dans l'étape de procédé d) étant chauffé à une température dans la plage de 80 °C à 160 °C.

7. Procédé selon l'une quelconque des étapes de procédé 1 à 6,
l'introduction de CO dans l'étape de procédé c) étant réalisée de telle manière, que la réaction a lieu à une pression de CO dans la plage de 20 bars à 60 bars.
